# EUROPEAN PATENT APPLICATION

(11) **EP 4 589 056 A1**
(43) Date of publication of application: **23.07.2025**
(21) Application number: 24210073.3
(22) Date of filing: 31.10.2024
(51) Int. Cl.: C30B 23/06, C30B 33/06, C30B 23/02, C30B 29/36

(54) **SIC INGOT AND METHOD FOR MANUFACTURING SIC SUBSTRATE**

(30) Priority: 28.12.2023 JP 2023223451; 28.12.2023 JP 2023223189; 28.12.2023 JP 2023223461; 25.10.2024 JP 2024188125; 25.10.2024 JP 2024188186; 25.10.2024 JP 2024188277
(71) Applicant: Resonac Corporation, Tokyo 105-7325 (JP)
(72) Inventor: Nagahata, Yukio, Tokyo, 105-7325 (JP)
(74) Representative: Strehl & Partner mbB

(57) **Abstract**

This SiC ingot includes a facet, wherein, when a diameter of the SiC ingot is represented as D and in a plan view in a crystal growth direction, a virtual rectangle that surrounds the facet with a minimum area and has a first side parallel to a <11-20> direction and a second side parallel to a <1-100> direction is drawn, and a length of the first side of the virtual rectangle is represented as Lx, Lx/D < 0.3 is satisfied at a first end which is a terminal of the crystal growth direction.

## Description

### BACKGROUND OF THE INVENTION

### Field of the Invention

The present invention relates to a SiC ingot and a method for manufacturing a SiC substrate.

The present application claims priority on Japanese Patent Application No. 2023-223189 filed on December 28, 2023, Japanese Patent Application No. 2023-223451 filed on December 28, 2023, Japanese Patent Application No. 2023-223461 filed on December 28, 2023, Japanese Patent Application No. 2024-188125 filed on October 25, 2024, Japanese Patent Application No. 2024-188186 filed on October 25, 2024, Japanese Patent Application No. 2024-188277 filed on October 25, 2024, the content of which is incorporated herein by reference.

### Description of Related Art

Silicon carbide (SiC) has a dielectric breakdown field one order of magnitude larger than silicon (Si) and a band gap three times larger than silicon (Si). In addition, silicon carbide (SiC) has properties such as a thermal conductivity about three times higher than silicon (Si). For this reason, silicon carbide (SiC) is expected to be applied to power devices, high frequency devices, high temperature operating devices, and the like. For this reason, in recent years, SiC epitaxial wafers have come to be used for the above-described semiconductor devices.

A SiC epitaxial wafer is obtained by stacking a SiC epitaxial layer on the surface of a SiC substrate. Hereinafter, the substrate before the SiC epitaxial layer is stacked thereon is referred to as a SiC substrate, and the substrate after the SiC epitaxial layer is stacked thereon is referred to as a SiC epitaxial wafer. The SiC substrate is cut out from a SiC ingot.

For example, as described in Patent Document 1, when a SiC ingot is produced, facets are formed in the SiC ingot. During the crystal growth of the SiC ingot, a part of a crystal growth surface becomes parallel to a c plane, and a surface parallel to the c plane is exposed on the crystal growth surface. This surface parallel to the c plane has a different pattern in crystal growth from other crystal growth surfaces on which step-flow growth is performed. A portion obtained through the crystal growth in a different pattern from a portion obtained through the step-flow growth is a facet.

### [Patent Documents]

[Patent Document 1] Japanese Patent No. 6050053

### SUMMARY OF THE INVENTION

In recent years, laser processing of a SiC ingot has been carried out. For example, cracks are created in a SiC ingot with a laser, and a SiC substrate is cut out from the SiC ingot. The optimum value of an output of the laser varies depending on the resistance value of a SiC single crystal. If the laser output is low, sufficient cracks may not be generated, whereas if the laser output is high, a processed surface may become rough. The facet has a lower resistance value than the portion obtained through the step-flow growth.

If the facet is present in the SiC ingot, it is necessary to modify the laser output. There is a problem that the greater the number of times that the laser output is changed, the lower the throughput of the processing.

The present invention has been made in consideration of the above-described problems, and an object of the present invention is to provide a SiC ingot which is easily laser-processed during laser processing and a method for manufacturing a SiC substrate using the SiC ingot.

The present inventors have found that the shape of the facet is controlled so as to reduce the number of times of changes in the laser output. The present invention provides the following solutions to solve the above problems.
(1) A SiC ingot according to a first aspect includes a facet. In this SiC ingot, when a diameter of the SiC ingot is represented as D and in a plan view in a crystal growth direction, a virtual rectangle that surrounds the facet with a minimum area and has a first side parallel to a <11-20> direction and a second side parallel to a <1-100> direction is drawn, and a length of the first side of the virtual rectangle is represented as Lx, Lx/D < 0.3 is satisfied at a first end which is a terminal of the crystal growth direction.
(2) In the SiC ingot according to the above aspect (1), 0.05 < Lx/D < 0.3 may be satisfied at the first end.
(3) In the SiC ingot according to the above aspect (1) or (2), Lx/D ≤ 0.2 may be satisfied at the first end.
(4) In the SiC ingot according to any one of the above aspects (1) to (3), Lx/D ≤ 0.1 may be satisfied at the first end.
(5) In the SiC ingot according to any one of the above aspects (1) to (4), Lx/D < 0.3 may be satisfied at a second end opposite to the first end.
(6) In the SiC ingot according to any one of the above aspects (1) to (5), 0.05 < Lx/D < 0.3 may be satisfied at the second end.
(7) In the SiC ingot according to any one of the above aspects (1) to (6), Lx/D < 0.3 may be satisfied in one cut surface that intersects with the crystal growth direction within a range of 90° ± 1°.
(8) In the SiC ingot according to any one of the above aspects (1) to (7), Lx/D < 0.3 may be satisfied in two or more cut surfaces that intersect with the crystal growth direction within a range of 90° ± 1°.
(9) In the SiC ingot according to any one of the above aspects (1) to (8), Lx/D < 0.3 may be satisfied in five or more cut surfaces that intersect with the crystal growth direction within a range of 90° ± 1°.
(10) In the SiC ingot according to any one of the above aspects (1) to (9), Lx/D < 0.3 may be satisfied in any cut surface that intersects with the crystal growth direction within a range of 90° ± 1°.
(11) In the SiC ingot according to any one of the above aspects (1) to (10), the SiC ingot may include a portion having an offset angle of 3.5° or more and 4.5° or less with respect to a {0001 } plane.
(12) In the SiC ingot according to any one of the above aspects (1) to (11), a height in the crystal growth direction may be 20 mm or more.
(13) In the SiC ingot according to any one of the above aspects (1) to (12), a diameter may be 145 mm or more.
(14) In the SiC ingot according to any one of the above aspects (1) to (12), a diameter may be 195 mm or more.
(15) In the SiC ingot according to any one of the above aspects (1) to (14), when a length of the second side of the virtual rectangle is represented as Ly, 0.5 > Ly/D may be satisfied at the first end.
(16) In the SiC ingot according to any one of the above aspects (1) to (15), when a length of the second side of the virtual rectangle is represented as Ly, 0.5 > Ly/D may be satisfied in any cut surface that intersects with the crystal growth direction within a range of 90° ± 1°.
(17) A method for manufacturing a SiC substrate according to a second aspect includes: a step of producing the SiC ingot according to any one of the above aspects (1) to (16); and a step of slicing the SiC ingot.
(18) A method for manufacturing a SiC substrate according to a third aspect includes: a step of preparing the SiC ingot according to any one of the above aspects (1) to (16); and a step of slicing the SiC ingot.

The SiC ingot according to the above aspects is easily processed during laser processing. In addition, the method for manufacturing a SiC substrate according to the above aspect has excellent production efficiency.

### BRIEF DESCRIPTION OF THE DRAWINGS

FIG. 1 is a perspective view of a SiC ingot according to the present embodiment.
FIG. 2 is a cross-sectional view of the SiC ingot according to the present embodiment.
FIG. 3 is a plan view of a cut surface obtained by cutting the SiC ingot according to the present embodiment.
FIG. 4 is a plan view of a cut surface obtained by cutting a SiC ingot according to a comparative example.
FIG. 5 is a plan view of the cut surface obtained by cutting the SiC ingot according to the comparative example.
FIG. 6 is a cross-sectional view of an example of an apparatus for manufacturing the SiC ingot according to the present embodiment.
FIG. 7 is a cross-sectional view of another example of the apparatus for manufacturing the SiC ingot according to the present embodiment.

### DETAILED DESCRIPTION OF THE INVENTION

Hereinafter, a SiC ingot and the like according to the present embodiment will be described in detail with reference to the drawings as appropriate. In the drawings which will be used in the following description, featured portions may be enlarged for convenience in order to make the features of the present embodiment easy to understand, and the dimensional ratios of constituent elements may be different from the actual ones. The materials, dimensions, and the like which will be exemplified in the following description are examples, and the present invention is not limited thereto and can be carried out with appropriate modifications without changing the features of the present invention.

In the present specification, an individual orientation is indicated by [ ], a collective orientation is indicated by < >, an individual plane is indicated by ( ), and a collective plane is indicated by { }. For negative indices, a "-" (bar) is customarily placed above the number in crystallography, but in the present specification, a negative sign is placed before the number.

In addition, the notation "a ± b" indicates the range of "a - b" or more and "a + b" or less.

First, directions are defined as follows. A crystal growth direction of a SiC ingot 10 is defined as a Z direction. The Z direction is a height direction of the SiC ingot 10 in a cylindrical shape. One direction in a plane orthogonal to the Z direction is defined as an X direction. The X direction is, for example, a <11-20> direction. In addition, in a plane orthogonal to the Z direction, a direction orthogonal to the X direction is defined as a Y direction. The Y direction is, for example, a <1-100> direction.

### "SiC ingot"

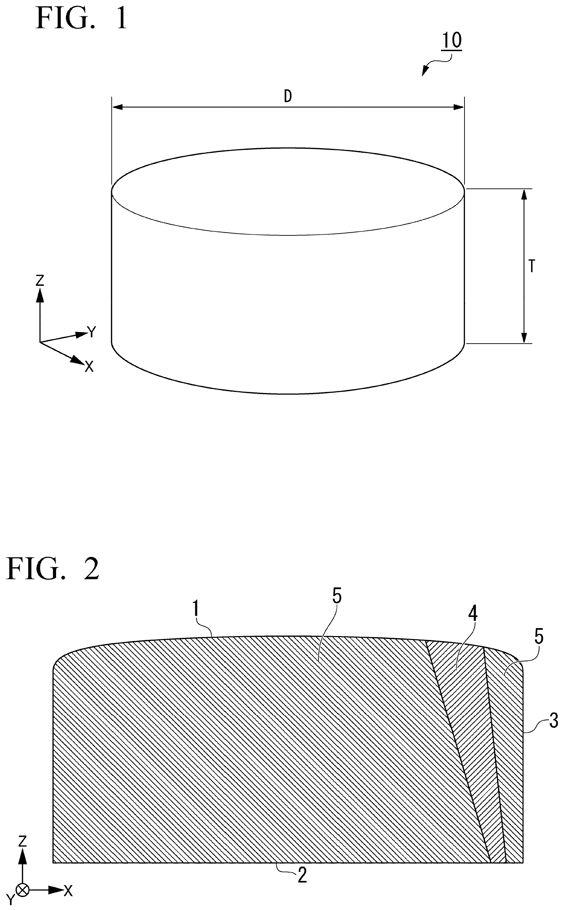

FIG. 1 is a perspective view of the SiC ingot 10 according to the present embodiment. The SiC ingot 10 is a cylindrical single crystal of SiC. The SiC ingot 10 is processed into a cylindrical shape such that a SiC substrate can be cut out therefrom.

The diameter D of the SiC ingot 10 is, for example, 145 mm or more, preferably 149 mm or more, more preferably 155 mm or less, and even more preferably 151 mm or less. The diameter D of the SiC ingot 10 is, for example, 195 mm or more, preferably 199 mm or more, more preferably 205 mm or less, and further preferably 201 mm or less. The SiC ingot 10 may be, for example, one from which a 6-inch substrate can be acquired, or one from which an 8-inch substrate can be acquired. The diameter D of the SiC ingot 10 approximately corresponds to the diameter of the SiC substrate after processing.

The thickness (the height in the crystal growth direction) T of the SiC ingot 10 is, for example, 20 mm or more, preferably 30 mm or more, more preferably 40 mm or more, further preferably 50 mm or more, and particularly preferably 60 mm or more. The thickness T of the SiC ingot 10 may be 100 mm or more. The thicker the SiC ingot 10, the more SiC substrates can be acquired. In addition, the thickness T of the SiC ingot 10 may be 300 mm or less.

The SiC ingot 10 includes, for example, a first end 1, a second end 2, and a side surface 3. The first end 1 is a terminal in the crystal growth direction and is, for example, a (000-1) plane or a plane inclined by an offset angle from the (000-1) plane. The second end 2 is an end portion opposite to the first end 1 and is, for example, a

(0001) plane or a plane inclined by an offset angle from the (0001) plane. The first end 1 and the second end 2 are the bottom surface and the top surface of a cylinder. The side surface 3 is a surface that connects the first end 1 and the second end 2 to each other and is a side surface of the cylinder.

FIG. 2 is a cross-sectional view of the SiC ingot 10 according to the present embodiment. The SiC ingot 10 includes a facet 4 and a step-flow growth region 5.

The SiC ingot 10 is obtained by performing the crystal growth on a SiC seed crystal. In order to suppress the occurrence of heterogeneous polymorphs, a seed crystal having an offset angle relative to the {0001} plane is often used. The offset angle is, for example, 3.5° or more and 4.5° or less, and is preferably 4°. By performing step-flow growth of SiC on the seed crystal, the occurrence of heterogeneous polymorphs can be suppressed. Even in a case in which the SiC ingot is obtained through the step-flow growth, a part of a crystal growth surface becomes parallel to the (0001) plane, and a surface parallel to the (0001) plane is exposed on the crystal growth surface. On the surface parallel to the (0001) plane, crystals grow perpendicular to the (0001) plane, and thus the step-flow growth is not performed.

The facet 4 is a region in which the crystals have grown perpendicular to the (0001) plane. The step-flow growth region 5 is a region in which the crystals have grown through the step-flow growth. The step-flow growth region 5 has an offset angle of, for example, 3.5° or more and 4.5° or less with respect to the {0001} plane.

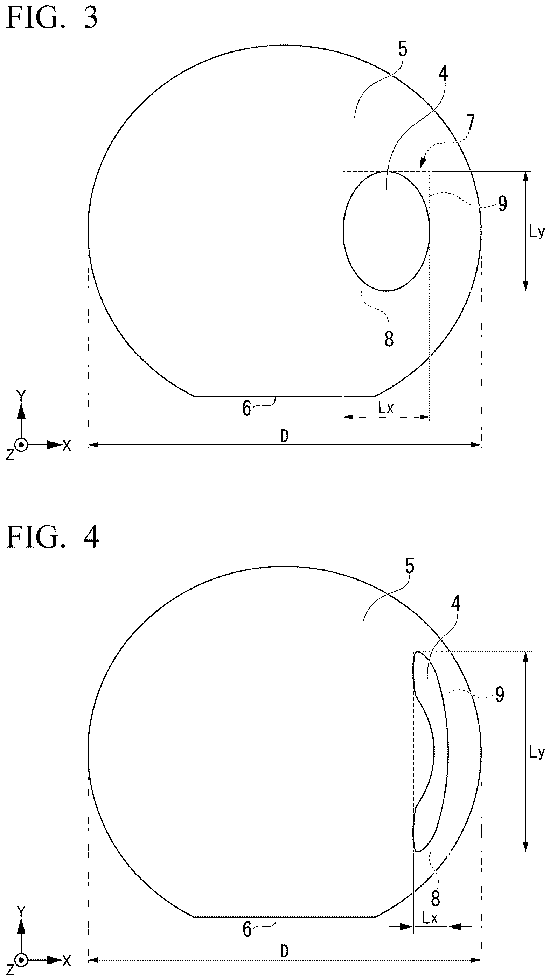

FIG. 3 is a plan view of a cut surface obtained by cutting the SiC ingot 10 according to the present embodiment along a plane approximately orthogonal to the Z direction. "Approximately orthogonal" means that the plane intersects with the Z direction within the range of 90° ± 1°. An XY plane of the SiC ingot 10 is approximately circular. The SiC ingot 10 may have an orientation flat 6 or a notch for identifying the direction of a crystal axis.

As shown in FIG. 3, in a plan view in the Z direction, the facet 4 and the step-flow growth region 5 have different colors, and a boundary between them can be visually observed. This is because the facet 4 and the step-flow growth region 5 have different patterns in crystal growth. The facet 4 is visually observed as a region darker in color than the step-flow growth region 5.

The boundary between the facet 4 and the step-flow growth region 5 can be determined visually, but may also be determined by the following procedure. First, an image of a cut surface to be measured is acquired. For example, the image is acquired by polishing both surfaces of the substrate and using a scanner. As the scanner, for example, a flatbed scanner manufactured by Canon Inc. can be used. The image may be acquired using a digital camera. Next, the acquired image is converted into an HLS color space consisting of hue, luminance, and saturation, and the luminance is calculated. Then, in the image converted into a luminance distribution, a circle of pixels with a radius X is drawn with an arbitrary pixel as the center of the circle. In a case in which there are pixels within this circle with a luminance difference from the central pixel of Y or more, the pixel at the center of the circle becomes a pixel that is a facet candidate. In a case in which there are no pixels within this circle with a luminance difference from the central pixel of Y or more, the pixel at the center of the circle becomes a pixel that is not a facet candidate. A similar treatment is then performed on all pixels of the image, and the pixels are classified as either pixels that are facet candidates or pixels that are not facet candidates. Then, the boundary between the pixels that are facet candidates and the pixels that are not facet candidates is detected, and the inside of this region becomes a facet. Among the pixels that are facet candidates, pixels that are isolated from other pixels that are facet candidates can be determined not to be facet candidates. The radius X of the circle and the luminance difference Y are set according to the size and number of pixels of the image. These settings involve setting values that do not cause a large discrepancy between the visual observation result and the determination result. For example, in a case in which a 640 pixel × 480 pixel image including a 150 mm-diameter wafer is used, the radius X is set to 9 pixels and the luminance difference Y is set to 4 W·sr⁻¹·m⁻².

It is assumed that there is a virtual rectangle 7 that surrounds the facet 4 with a minimum area. The virtual rectangle 7 is a rectangle having a first side 8 and a second side 9. The first side 8 is a side parallel to the <11-20> direction (the X direction). The second side 9 is a side parallel to the <1-100> direction.

The length Lx of the first side 8 satisfies Lx/D < 0.3 in relationship with the diameter D of the SiC ingot 10. Lx/D preferably satisfies Lx/D ≤ 0.25, more preferably satisfies Lx/D ≤ 0.2, further preferably satisfies Lx/D ≤ 0.15, and particularly preferably satisfies Lx/D ≤ 0.1. In addition, Lx/D may satisfy 0.05 < Lx/D, preferably satisfies 0.06 < Lx/D, and more preferably satisfies 0.07 < Lx/D. It is preferable that this relationship be satisfied at the first end 1 of the SiC ingot 10. In addition, it is more preferable that this relationship be also satisfied at the second end 2 of the SiC ingot 10. In addition, it is further preferable that the relationship of Lx/D be also satisfied in the cut surface of the SiC ingot 10 which is approximately orthogonal to the Z direction at one position in the Z direction. It is further preferable that the relationship of Lx/D be also satisfied in each cut surface obtained by cutting the SiC ingot 10 along a plane approximately orthogonal to the Z direction at two or more positions in the Z direction. It is further preferable that the relationship of Lx/D be also satisfied in each cut surface obtained by cutting the SiC ingot 10 along a plane approximately orthogonal to the Z direction at five or more positions in the Z direction. It is further preferable that the relationship of Lx/D be also satisfied in a cut surface obtained by cutting the SiC ingot 10 along a plane approximately orthogonal to the Z direction at any position in the Z direction.

When the SiC ingot 10 satisfies the above-described relationship, the efficiency of acquiring a SiC substrate using a laser is improved. When acquiring a SiC substrate using a laser, a treatment of irradiating the SiC ingot with the laser while scanning the SiC ingot with the laser in the Y direction is repeated while moving the laser in the X direction. If the facet 4 is encountered during the laser scanning in the Y direction, a change in laser output is required to create a suitable crack. This is because the facet 4 and the step-flow growth region 5 have different resistance values. If the width of the facet 4 in the X direction is narrow, the width of a portion at which the laser output needs to be changed during the laser scanning in the Y direction becomes narrow. In other words, when Lx/D < 0.3 is satisfied, the proportion of the portion at which the laser scanning can be performed without changing the laser output is large, and the SiC substrate can be efficiently cut out. In addition, this tendency becomes more noticeable as the value of Lx/D becomes smaller.

In addition, FIG. 4 is a plan view of a cut surface obtained by cutting a SiC ingot according to a comparative example along a plane approximately orthogonal to the Z direction. In the example shown in FIG. 4, 0.05 ≥ Lx/D. If the value of Lx/D is too small, the facet 4 will often be curved and become a crescent shape, as shown in FIG. 4. If the facet 4 becomes a crescent shape, a plurality of changes in the laser output may be required during one scan of the laser in the Y direction. In this case, the number of times that the laser output is changed in order to create an appropriate crack is increased, and thus the throughput for acquiring a SiC substrate is deteriorated.

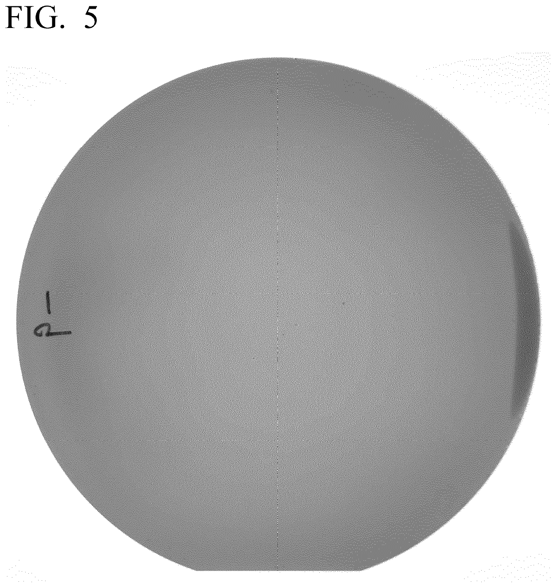

For example, FIG. 5 is a plan image of a SiC substrate cut out from a SiC ingot that satisfies 0.05 ≥ Lx/D. As shown in FIG. 5, the curvature of the facet 4 with a small value of Lx/D can be checked.

The length Lx of the first side 8 is preferably 30 mm or less, more preferably 25 mm or less, further preferably 20 mm or less, further preferably 15 mm or less, and further preferably 10 mm or less.

In addition, the length Ly of the second side 9 preferably satisfies Ly/D < 0.5, more preferably satisfies Ly/D ≤ 0.45, and further preferably satisfies Ly/D ≤ 0.4 in relationship with the diameter D of the SiC ingot 10. It is preferable that this relationship be satisfied at the first end 1 of the SiC ingot 10. In addition, it is more preferable that this relationship be also satisfied at the second end 2 of the SiC ingot 10, and it is further preferable that this relationship be also satisfied in a cut surface obtained by cutting the SiC ingot 10 along a plane approximately orthogonal to the Z direction at one position in the Z direction. In addition, it is further preferable that this relationship be also satisfied in each cut surface obtained by cutting the SiC ingot 10 along a plane approximately orthogonal to the Z direction at two or more positions in the Z direction. In addition, it is further preferable that this relationship be also satisfied in each cut surface obtained by cutting the SiC ingot 10 along a plane approximately orthogonal to the Z direction at five or more positions in the Z direction. In addition, it is further preferable that the relationship of Lx/D be also satisfied in a cut surface obtained by cutting the SiC ingot 10 along a plane approximately orthogonal to the Z direction at any position in the Z direction. In addition, Ly/D may be greater than 0.01.

If the value of Ly/D is large, it is highly likely that the facet 4 will be curved and become a crescent shape.

By making the facet 4 elliptical in a plan view, the number of times that the laser output is changed can be reduced.

### "Method for manufacturing SiC ingot"

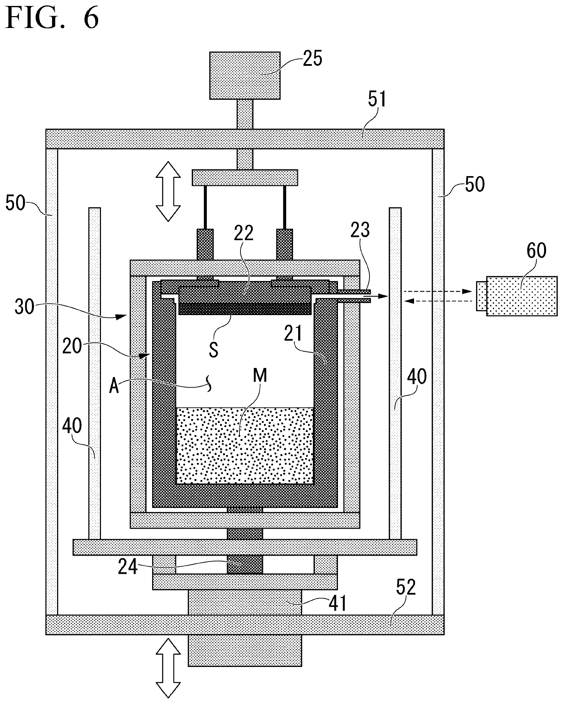

Next, a method for manufacturing the SiC ingot 10 according to the present embodiment will be described. FIG. 6 is a cross-sectional view of an example of an apparatus for manufacturing the SiC ingot 10 according to the present embodiment. The apparatus for manufacturing the SiC ingot 10 includes a crucible 20, a heat insulating material 30, a liner quartz tube 40, a quartz tube 50, and a reflectance measuring instrument 60.

The crucible 20 is made of, for example, graphite. The crucible 20 includes an accommodation portion 21 and a lid 22. A gas discharge path 23 is formed between the accommodation portion 21 and the lid 22. The gas discharge path 23 is provided at least at one location on the outer side surface of the crucible 20 and may be provided at a plurality of locations. In addition, the gas discharge path 23 may be provided in a ring shape around the outer side surface of the crucible 20 between the accommodation portion 21 and the lid 22. The accommodation portion 21 is supported by and fixed to a support 24. The lid 22 is suspended by a suspension member 25 and can be moved up and down. A distance between the accommodation portion 21 and the lid 22 can be freely changed by moving the suspension member 25 up and down. That is, the width of the gas discharge path 23 in the Z direction can be freely designed.

A seed crystal S and a SiC raw material M are disposed in the film deposition space A in the crucible 20. Gas sublimated from the SiC raw material M is recrystallized on the surface of the seed crystal S, and thus the crystal growth of the SiC ingot 10 is caused. A residual gas in the film deposition space A is discharged to the outside of the crucible 20 through the gas discharge path 23.

The atmospheric pressure in the film deposition space A during the crystal growth is set to be more than 0.3 Torr and less than 10 Torr. If the pressure in the film deposition space A is too low, a dopant element cannot be sufficiently incorporated into the crystal, and the resistivity of the SiC ingot 10 becomes high. The resistivity of the SiC ingot 10 is a parameter that affects the laser processing. In addition, if the pressure in the film deposition space A is too high, a sufficient amount of a sublimation gas is not generated from the SiC source material M; and as a result, productivity decreases.

The bulk density of the graphite forming the side surface of the crucible 20 is set to be more than 1.75 g/cm³ and less than 2.00 g/cm³. A part of the residual gas in the film deposition space A permeates through the crucible 20 and escapes to the outside. If the bulk density of the graphite forming the crucible 20 is low, a large amount of the residual gas will be discharged from portions other than the gas discharge path 23. The residual gas discharged from positions other than the gas discharge path 23 deteriorates the heat insulating material 30. On the other hand, if the bulk density of the graphite forming the crucible 20 is too high, the amount of thermal conductivity of the graphite becomes too large, and thus it is not possible to achieve an appropriate temperature distribution inside the crucible 20.

The volume G of the graphite forming the crucible 20 is set to be 1.5 to 4.0 times the volume F based on the diameter of the seed crystal S. The volume F corresponds to the inner volume of the crucible 20 and can be calculated by π × d³/2, where d is the diameter of the seed crystal S. If the volume of the graphite is small, the amount of the residual gas that permeates the crucible 20 increases, and this causes the heat insulating material 30 to deteriorate. If the volume of the graphite is too large, the thickness of the graphite becomes too large, and thus it is not possible to achieve an appropriate temperature distribution inside the crucible 20.

The heat insulating material 30 covers the periphery of the crucible 20. The volume of the heat insulating material 30 is more than one time and less than three times the volume of the crucible 20. The volume of the heat insulating material 30 is the volume of the heat insulating material 30 itself and is the volume of the area surrounded by the outer surface and the inner surface of the heat insulating material 30. The volume of the crucible 20 is the volume of the inside surrounded by the outer surface of the crucible. By setting the volume of the heat insulating material 30 within this range, it is possible to achieve an appropriate temperature distribution inside the crucible 20. The temperature distribution inside the crucible 20 affects the formation of the facet 4.

In addition, the outer diameter of the heat insulating material 30 is set to be 1.2 times or more and 1.5 times or less the outer diameter of the crucible 20. The heat insulating material 30 located on the side surface of the crucible 20 is in direct contact with the crucible 20 which generates heat due to induction heating, and thus heat insulating performance is easily deteriorated. When the heat insulating material 30 within the above range has a sufficient width, it is possible to achieve an appropriate temperature distribution inside the crucible 20 while preventing deterioration of the heat insulating material 30.

The liner quartz tube 40 is positioned around the heat insulating material 30. At least a part of the liner quartz tube 40 faces the end portion of the gas discharge path 23. The residual gas discharged from the gas discharge path 23 is applied onto the liner quartz tube 40. The liner quartz tube 40 is supported by a support 41. As the support 41 moves up and down, the liner quartz tube 40 also moves up and down. During the crystal growth, the liner quartz tube 40 is moved downward at a constant speed. The residual gas discharged from the gas discharge path 23 is blown onto the liner quartz tube 40, is adhered to the inner wall of the liner quartz tube 40, and is solidified. The reflectance of the liner quartz tube 40 increases as the residual gas is solidified.

The quartz tube 50 surrounds the crucible 20, the heat insulating material 30, and the liner quartz tube 40. The quartz tube 50 is covered with an upper lid 51 and a lower lid 52. The quartz tube 50 controls the atmosphere in the film deposition space A.

The reflectance measuring instrument 60 measures the reflectance of the portion of the liner quartz tube 40 to which the residual gas is applied. The reflectance of this portion increases as the amount of the gas discharged from the gas discharge path 23 increases.

The reflectance measuring instrument 60 measures the amount of the gas discharged from the gas discharge path 23 by measuring the reflectance of the portion of the liner quartz tube 40 to which the gas is applied. During the crystal growth, the liner quartz tube 40 is moved downward at a constant speed. For this reason, the position in the liner quartz tube 40 to which the gas is applied is constantly changing. If the amount of the gas discharged from the gas discharge path 23 is constant, the reflectance of the portion of the liner quartz tube 40 of which the reflectance is measured by the reflectance measuring instrument 60 is constant.

When producing the desired SiC ingot 10, the flow of the sublimation gas in the film deposition space A needs to be kept constant. When the amount of the gas discharged from the gas discharge path 23 changes, the flow of the sublimation gas in the film deposition space A changes.

In the apparatus for manufacturing the SiC ingot 10 according to the present embodiment, when the reflectance of the portion which is measured by the reflectance measuring instrument 60 changes, the width of the gas discharge path 23 is changed by moving the suspension member 25 up and down. For example, in a case in which the reflectance of the portion which is measured by the reflectance measuring instrument 60 decreases, the amount of the gas discharged from the gas discharge path 23 is small, and thus the width of the gas discharge path 23 is to be increased. For example, in a case in which the reflectance of the portion which is measured by the reflectance measuring instrument 60 changes to a high value, the amount of the gas discharged from the gas discharge path 23 is large, and thus the width of the gas discharge path 23 is to be reduced.

The apparatus for manufacturing the SiC ingot 10 according to the present embodiment can also control the flow of the sublimation gas within the film deposition space A by changing the width of the gas discharge path 23 as described above. In this manner, by controlling the temperature distribution and the flow of the sublimation gas within the film deposition space A, the SiC ingot according to the present embodiment can be produced.

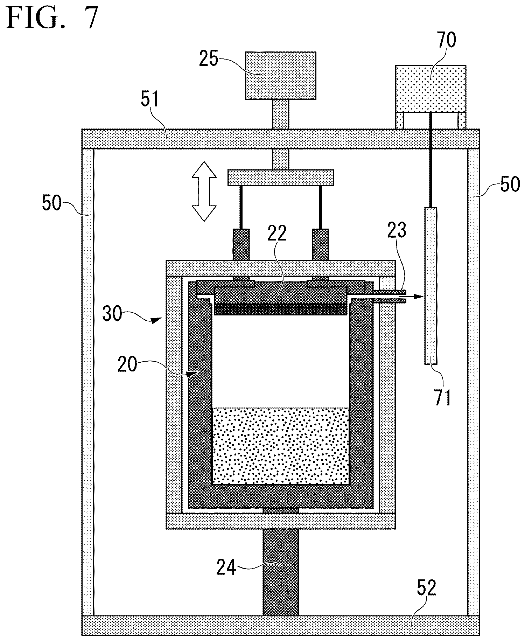

In addition, FIG. 7 is a cross-sectional view of another example of the apparatus for manufacturing the SiC ingot 10 according to the present embodiment. The apparatus for manufacturing the SiC ingot 10 shown in FIG. 7 includes a crucible 20, a heat insulating material 30, a quartz tube 50, a weight measuring instrument 70, and an evaluation substrate 71. The apparatus for manufacturing the SiC ingot 10 shown in FIG. 7 is provided with the evaluation substrate 71 instead of the liner quartz tube 40, and the weight measuring instrument 70 instead of the reflectance measuring instrument 60. The configurations of the crucible 20, the heat insulating material 30, and the quartz tube 50 are the same as those shown in FIG. 6.

In the apparatus for manufacturing the SiC ingot 10 shown in FIG. 7, the residual gas discharged from the gas discharge path 23 is applied to the evaluation substrate 71. The residual gas applied to the evaluation substrate 71 is solidified on the surface of the evaluation substrate 71. The weight of the evaluation substrate 71 is increased as the residual gas is solidified.

The weight measuring instrument 70 measures the weight of the evaluation substrate 71. If the amount of the gas discharged from the gas discharge path 23 is constant, the rate of the weight increase of the evaluation substrate 71 is constant. In contrast, when the amount of the gas discharged from the gas discharge path 23 increases, the rate of the weight increase of the evaluation substrate 71 increases, and when the amount of the gas discharged from the gas discharge path 23 decreases, the rate of the weight increase of the evaluation substrate 71 decreases. In the apparatus for manufacturing the SiC ingot 10 shown in FIG. 7, the amount of the gas discharged from the gas discharge path 23 is evaluated on the basis of the change in the rate of the weight increase of the evaluation substrate 71.

The apparatus for manufacturing the SiC ingot 10 shown in FIG. 7 can also control the flow of the sublimation gas within the film deposition space A by changing the width of the gas discharge path 23 such that the amount of the gas discharged from the gas discharge path 23 is constant. In this manner, by controlling the temperature distribution and the flow of the sublimation gas within the film deposition space A, the SiC ingot according to the present embodiment can be produced.

As an example of a method for evaluating the amount of the gas discharged from the gas discharge path 23, an example in which a change in the reflectance or a change in the weight is used is shown, but the physical quantity for evaluating the amount of the gas discharged from the gas discharge path 23 is not limited to these.

As described above, in the SiC ingot 10 according to the present embodiment, the shape of the facet 4 is controlled. In the SiC ingot 10 according to the present embodiment, the number of times that a scanning direction of the laser and the facet 4 intersects with each other is controlled to be reduced, and thus the number of times that the laser output is changed during the laser processing can be reduced. For this reason, in the SiC ingot 10 according to the present embodiment, it is possible to reduce the number of times that the laser output is changed when cutting out the SiC substrate using the laser, and it is possible to efficiently produce the SiC substrate.

### "Method for manufacturing SiC substrate"

A method for manufacturing a SiC substrate of a first embodiment includes a step of manufacturing the SiC ingot 10 of the embodiment described above by any of the methods described above, and a step of slicing the SiC ingot 10.

As the step of slicing the SiC ingot, for example, a method in which the SiC ingot is processed with a laser to create cracks and cut out the SiC substrates can be used. The SiC ingot 10 may be processed into a cylindrical shape before the step of slicing the SiC ingot 10.

The SiC substrate cut out from the SiC ingot 10 according to the present embodiment is highly likely to satisfy Lx/D < 0.3. For example, in a case in which the SiC ingot 10 satisfies Lx/D < 0.3 in any cut surface orthogonal to the crystal growth direction, the SiC substrate cut out from this SiC ingot 10 satisfies Lx/D < 0.3. In other words, in a case in which all of the plurality of SiC substrates cut out from the same SiC ingot satisfy Lx/D < 0.3, the SiC ingot before cutting out can be said to correspond to the SiC ingot of the present embodiment. For example, in a case in which all of the SiC substrates having a thickness of 0.5 mm cut out from the same SiC ingot satisfy Lx/D < 0.3, the SiC ingot before cutting out has a thickness of 20 mm or more and can be said to correspond to the SiC ingot of the present embodiment.

A method for manufacturing a SiC substrate of a second embodiment includes a step of preparing the SiC ingot 10 of the embodiment described above, and a step of slicing the SiC ingot 10.

The step of preparing the SiC ingot 10 may include obtaining the SiC ingot 10 of the above-mentioned embodiment from other companies, and the SiC ingot 10 may be a boule, provided that the boule meets the features of the SiC ingot 10 of the above-mentioned embodiment.

The step of slicing the SiC ingot 10 is the same as that in the first embodiment. The SiC ingot 10 may be processed into a cylindrical shape before the step of slicing the SiC ingot 10.

As described above, the preferable embodiments of the present invention have been described in detail, the present invention is not limited to specific embodiments, and various modifications and changes can be made within the scope of the features of the present invention described in the claims.

### [Examples]

### "Example 1"

Using the apparatus for manufacturing a SiC ingot shown in FIG. 6, a SiC ingot having a diameter of 150 mm was produced while controlling the temperature distribution and the flow of the sublimation gas in the film deposition space A. The SiC ingot of Example 1 had a facet at a first end which was a terminal in the crystal growth direction.

The length Lx of a first side of a virtual rectangle that surrounded the facet with the minimum area was 15 mm. That is, the SiC ingot of Example 1 satisfied Lx/D = 0.1.

Next, the SiC ingot of Example 1 was laser processed to cut out a SiC substrate. The scan pitch of the laser was set to 200 µm, the feed speed of the laser scan was set to 200 mm/sec, the number of scans was set to 1, the acceleration/deceleration time was set to 0.1 sec, and the inter-line movement time was set to 0.1 sec. The acceleration/deceleration time was the time required to accelerate and decelerate when scanning with the laser in one direction and then scanning with the laser in an opposite direction. The inter-line movement time indicated the time required for movement in the X direction in a treatment in which laser scanning in the Y direction and the movement in the X direction were repeated. In addition, when the laser crossed the boundary between the facet and the step-flow growth region, the scan was stopped for 0.5 seconds to change the laser output.

The process time required to cut out one SiC substrate from the SiC ingot of Example 1 was 11.1 minutes.

### "Example 2"

The SiC ingot having a diameter of 150 mm was produced in the same manner as Example 1 except that the pressure of the atmosphere in the film deposition space A was adjusted to a value which was different from that in Example 1. The SiC ingot of Example 2 had a facet at a first end which was a terminal in the crystal growth direction.

The length Lx of a first side of a virtual rectangle that surrounded the facet with the minimum area was 36 mm. That is, the SiC ingot of Example 2 satisfied Lx/D = 0.24.

Next, the SiC ingot of Example 2 was laser processed to cut out a SiC substrate. The conditions for the laser for cutting out the SiC substrate were the same as those in Example 1.

The process time required to cut out one SiC substrate from the SiC ingot of Example 2 was 12.9 minutes.

### "Comparative Example 1"

Without using the apparatus for manufacturing a SiC ingot shown in FIG. 6, the crystal growth of a SiC ingot was performed in a sealed crucible to produce a SiC ingot having a diameter of 150 mm. The SiC ingot of Comparative Example 1 included a facet at a first end which was a terminal in the crystal growth direction. The length Lx of a first side of a virtual rectangle that surrounded the facet with the minimum area was 60 mm. That is, the SiC ingot of Comparative Example 1 satisfied Lx/D = 0.4.

Next, the SiC ingot of Comparative Example 1 was laser processed to cut out a SiC substrate. The conditions for the laser for cutting out the SiC substrate were the same as those in Example 1.

The process time required to cut out one SiC substrate from the SiC ingot of Comparative Example 1 was 14.9 minutes.

The process time required to cut out the SiC substrate from each of the SiC ingots of Examples 1 and 2 was shorter than that required to cut out the SiC ingot of Comparative Example 1.

In Examples 1 and 2 and Comparative Example 1, the results for the SiC ingots with a diameter of 150 mm after processing have been shown so far. SiC ingots with a diameter of 200 mm after processing were also produced in the same manner, and a similar study was conducted for the SiC ingots with a diameter of 200 mm after processing. The same trend was observed in the case of 200 mm diameter as in the case of 150 mm diameter.

That is, the SiC ingot produced under the conditions of the embodiments satisfied Lx/D < 0.3. In contrast, the SiC ingot produced under the conditions that were out of the conditions of the embodiments satisfied Lx/D ≥ 0.3. In addition, the time required to cut out one SiC substrate from the SiC ingot produced under the conditions of the embodiments was shorter than the time required to cut out one SiC substrate from the SiC ingot produced under the conditions that were out of the conditions of the embodiments.

### EXPLANATION OF REFERENCES

1 First end
2 Second end
3 Side surface
4 Facet
5 Step-flow growth region
6 Orientation flat
7 Virtual rectangle
8 First side
9 Second side
10 SiC ingot
20 Crucible
21 Accommodation portion
22 Lid
23 Gas discharge path
24 Support
25 Suspension member
30 Heat insulating material
40 Liner quartz tube
41 Support
50 Quartz tube
51 Upper lid
52 Lower lid
60 Reflectance measuring instrument
70 Weight measuring instrument
71 Evaluation substrate

## Claims

1. A SiC ingot comprising a facet,
wherein, when a diameter of the SiC ingot is represented as D and
in a plan view in a crystal growth direction, a virtual rectangle that surrounds the facet with a minimum area and has a first side parallel to a <11-20> direction and a second side parallel to a <1-100> direction is drawn, and a length of the first side of the virtual rectangle is represented as Lx,
Lx/D < 0.3 is satisfied at a first end which is a terminal of the crystal growth direction.

2. The SiC ingot according to claim 1, wherein 0.05 < Lx/D < 0.3 is satisfied at the first end.

3. The SiC ingot according to claim 1 or 2, wherein Lx/D ≤ 0.2 is satisfied at the first end.

4. The SiC ingot according to any one of claims 1 to 3, wherein Lx/D ≤ 0.1 is satisfied at the first end.

5. The SiC ingot according to any one of claims 1 to 4, wherein Lx/D < 0.3 is satisfied at a second end opposite to the first end.

6. The SiC ingot according to any one of claims 1 to 5, wherein 0.05 < Lx/D < 0.3 is satisfied at the second end.

7. The SiC ingot according to any one of claims 1 to 6, wherein Lx/D < 0.3 is satisfied in one cut surface that intersects with the crystal growth direction within a range of 90° ± 1°.

8. The SiC ingot according to any one of claims 1 to 7, wherein Lx/D < 0.3 is satisfied in two or more cut surfaces that intersect with the crystal growth direction within a range of 90° ± 1°.

9. The SiC ingot according to any one of claims 1 to 8, wherein Lx/D < 0.3 is satisfied in five or more cut surfaces that intersect with the crystal growth direction within a range of 90° ± 1°.

10. The SiC ingot according to any one of claims 1 to 9, wherein Lx/D < 0.3 is satisfied in any cut surface that intersects with the crystal growth direction within a range of 90° ± 1°.

11. The SiC ingot according to any one of claims 1 to 10, wherein the SiC ingot comprises a portion having an offset angle of 3.5° or more and 4.5° or less with respect to a {0001 } plane.

12. The SiC ingot according to any one of claims 1 to 11, wherein a height in the crystal growth direction is 20 mm or more.

13. The SiC ingot according to any one of claims 1 to 12, wherein a diameter is 145 mm or more.

14. The SiC ingot according to any one of claims 1 to 12, wherein a diameter is 195 mm or more.

15. The SiC ingot according to any one of claims 1 to 14,
wherein, when a length of the second side of the virtual rectangle is represented as Ly,
0.5 > Ly/D is satisfied at the first end.

16. The SiC ingot according to any one of claims 1 to 15,
wherein, when a length of the second side of the virtual rectangle is represented as Ly,
0.5 > Ly/D is satisfied in any cut surface that intersects with the crystal growth direction within a range of 90° ± 1°.

17. A method for manufacturing a SiC substrate comprising:
a step of producing the SiC ingot according to any one of claims 1 to 16; and
a step of slicing the SiC ingot.

18. A method for manufacturing a SiC substrate comprising:
a step of preparing the SiC ingot according to any one of claims 1 to 16; and
a step of slicing the SiC ingot.
